# EUROPEAN PATENT APPLICATION

(11) **EP 4 206 267 A1**
(43) Date of publication of application: **05.07.2023**
(21) Application number: 21306957.8
(22) Date of filing: 29.12.2021
(51) Int. Cl.: C08J 11/12, C07C 67/333, C07C 69/54, C10B 53/07, C10G 1/02

(54) **PROCESS FOR RECYCLING CONTAMINATED POLYMERS**

(71) Applicant: ARKEMA FRANCE, 92700 Colombes (FR); Heathland B.V., 3565 AR Utrecht (NL)
(72) Inventor: DUBOIS, Jean-Luc, 92700 COLOMBES (FR); VAN DER HEIJDEN, Simon, Jan Josef, 3402SE IJSSELSTEIN (NL)
(74) Representative: Lavoix

(57) **Abstract**

The present invention concerns an improved depolymerization process of contaminated polymers such as PMMA, comprising the separation of contaminants from the resulting monomers, by using appropriate agents for trapping the contaminants.

## Description

The project leading to this application has received funding from the European Union's Horizon 2020 research and innovation programme under grant agreement No 820687.

The present invention concerns the field of plastics recycling by depolymerization.

More particularly, it concerns the recycling of plastics based on polymers, such as poly(methyl methacrylate) (PMMA) that are often contaminated by various additives. At the exit of a depolymerization reactor, such as a pyrolysis unit, the gas stream contains dusts, mist particles that comprise non-fully depolymerized polymer, oligomers and/or degraded polymers in addition to the desired monomers: these various ingredients may also include the contaminants initially present in the starting polymers.

For example, polyethylene is present together with PMMA as protection film. Polyethylene will degrade at higher temperature than PMMA, and therefore generates long chain oligomers such as high molecular weight waxes. These waxes can travel as mist particles or vapors in the unit.

The contaminants can also include polyethylene terephthalate (PET) which degrades into benzoic acid, terephthalic acid and dimethyl terephthalate, for example.

Further contaminants include EVA (Ethylene-Vinyl Acetate Copolymer), which is used as a PVC replacement in cast sheet production (PCT/NL2021/050687). EVA decomposes into acetic acid, which may generate corrosion, and a backbone which is a hydrocarbon polymeric structure. That polymeric structure will degrade also and generate a wide spectrum of oligomers of different chain lengths, which contribute to decrease the quality of the recovered crude MMA.

Other additives used in PMMA-based plastics include pigments. All kinds of pigments have been used through the ages and include some toxic metals/compounds that are no longer allowed today (or tomorrow), but still present in post-consumer/end-of-life products. Notably, such pigments include metals like cadmium, lead, chromium, selenium, mercury for example. Some PMMA grades still contain pigments incorporating these metals of high toxicity. In the course of the recycling of PMMA, they are released during the depolymerization process: although some can produce a solid residue and be trapped in a separator tank located between the depolymerization reactor and the condenser, some could still travel with the MMA vapors to the condenser. They will contribute to the color of the crude MMA and contaminate the crude MMA.

Further additives used in PMMA-based plastics include plasticizers, such as phthalates and other esters; cross linking agents such as methacrylate esters of ethylene glycol and other diols or other comonomers; waxes; flame retardants; brominated and halogenated compounds; polyaromatic hydrocarbons,.... The toxicity of such used additives presents serious threats to recycled products and residues generated in the depolymerization process.

US 8,304,573 concerns a fluidized bed depolymerization reactor and teaches to use solid particles for pyrolyzing resins. Calcium oxide, hydroxide or carbonate may be used when the (meth)acrylic resin to be depolymerized includes chlorinated compounds.

However, no option is offered to reduce the concentration of other types of contaminants.

Additionally, the disclosed fluidized bed reactor has the downside that the process consumes a large amount of energy, as the heat is first transferred to a sand in a combustion chamber in which carbon residues from the polymer, but also partially depolymerized PMMA and cracking gases would be burned together with additional fuel, in order to bring the sand to a high temperature, well above the depolymerization temperature. In addition, in order to fluidize the bed without using a high flux of inert gas, not only a mechanical agitation is necessary, but also the fluidization gas preferably contains recycled MMA to keep a high partial pressure of MMA in the condensation stage. Because of the high temperature of the hot sand, side reactions promoting the over cracking of the monomer are taking place, reducing the selectivity of the reaction. Furthermore, in this fluidized bed, the injection of molten polymer leads to sticky particles, which agglomerate and lead to zones in the reactor which are not properly fluidized and other zones where the gas velocity is increased leading to high bed instability and transport of solid particles outside of the reactor. In order to keep the selectivity of the process and a sufficient conversion of the PMMA, the productivity of the reactor is limited, meaning that the feeding rate in this type of reactor has to be reduced. Finally, in this process the sand has to circulate between a reactor where the depolymerization is taking place and a regenerator where the temperature of the sand has to be increased again.

Additionally, the fluidized bed would not be compatible with a solid additive: When a solid additive would be used in the process, such as calcium oxide, it would accumulate in the bed and would replace sand until its mechanical properties are sufficiently reduced by attrition. Then it would be carried away with the gasses and would accumulate in the condenser. In the case it is not carried away, it would completely substitute the sand in the fluidized bed which would lose its hydrodynamic characteristics.

Wu et al (proceedings of the 5th ISFR, 11-14 october 2009,89-95) report the addition of Ca(OH)2 for recycling waste plastics containing PET and PVC. The additive was used to reduce the corrosion and clogging issues caused by HCI generated from PVC, and benzoic acid produced from PET. Nevertheless, they do not suggest how to trap alternative contaminants. Further, the process generates a high amount of solid residue (20 wt %) and a low level of liquid "hydrocarbon oil" (51 wt %). In the process description of Figure 4 in the document, it appears clearly that the dechlorination process takes place before the pyrolysis. This step, then appears as an independent step in the process. In any case, the process is limited to the recycling of municipal waste plastics which are very rich in polyolefins (Polyethylene and Polypropylene) since these are the main plastics used in packaging besides PET. PET has other valorization routes and is usually sorted from the mix plastic wastes. So the process does not teach any specifics for the recycling of polymers like PMMA, PS and PHA for example.

There is therefore a need to provide an improved depolymerization processes that can reduce the concentration of contaminants in the monomers resulting from the depolymerization of polymers.

Unexpectedly, it has been discovered that such contaminants present in the polymers to be recycled, and/or generated during the depolymerization of said polymers, can be trapped and/or degraded through the depolymerization process and eventually be separated from the resulting monomers.
The side products generated during the depolymerization process including the contaminants (such as the solid residue from the cracking stage) can thus be directed to separate streams, in order to avoid to contaminate the condensed liquid, and to be submitted to a proper wastes management following the depolymerization process.

As a result, the concentration of the contaminants in the condensed monomers can be significantly reduced.

A problem to solve is therefore to find appropriate compounds that would be added with the plastics to be recycled, and that could convert the side products that are generated into less problematic and more stable compounds.

This problem has been solved by the present invention, which provides the addition of one or more trapping agent(s) which allow to separate/degrade the contaminants or degradation products generated during the depolymerization process.

Therefore, according to a first object, the present invention concerns a process for recycling plastics comprising one or more polymers contaminated by contaminants, where at least one contaminant is other than PET, PVC or their degradation products, said process comprising the steps of:
contacting at least one solid trapping agent with the contaminated polymers to be depolymerized ;
depolymerizing the polymers leading to corresponding monomers ;
whereby the non-PET and non-PVC contaminants interact with the solid trapping agents ; and
continuously extracting at least partially the non-PET and non-PVC contaminants associated with the solid trapping agent from the reaction mixture.

The present invention allows to trap contaminants, which would otherwise have been transferred with the resulting monomers in a condenser.

According to the invention, the contaminants interact with the trapping agents during the depolymerization process. The resulting product typically leads to solid residues. Consequently, the contaminants associated with the trapping agents may be directed to a separator configured to collect impurities such as dust, mist, waxes, char or oligomers. The collected material can then be directed to the appropriate disposal or recovery process.

As a result, the contaminants can be easily separated from the monomers that are recovered.

The term "plastics" typically refers to the materials that contain polymers as their main ingredient, together with optional additives. In the course of the recycling of the plastics, the additives may be undesired and are herein considered as contaminants as defined below.

The recycling of plastics as used herein comprises the depolymerization of the polymers which constitute said plastics.

The term "polymer" means either a copolymer or a homopolymer. A "copolymer" is a polymer grouping together several different monomer units and a "homopolymer" is a polymer grouping together identical monomer units.

The term « depolymerization » refers to the process for converting a polymer or polymers mixture into their constituting monomers or co-monomers.

The process of the invention is not restricted to particular polymers to be recycled.

According to an embodiment, suitable polymers may comprise poly(methyl methacrylate) (PMMA), polystyrene (PS), polyhydroxyalcanoates (PHA) which include poly-3-hydroxybutyrate, poly-3-hydroxy-butyrate-4-hydroxy-butyrate, poly-3-hydroxypropionate (also called polypropiolactone), polyhydroxybutyrate-valerate, polyhydroxyisobutyrate (also called poly-methyl-propiolactone).

According to a particular embodiment, PMMA is preferred.

PMMA refers to a thermoplastic homo- or copolymer of methyl methacrylate (MMA) that comprises at least 50%, preferably at least 60%, advantageously at least 70% and more advantageously at least 80% by weight of methyl methacrylate.

### PMMA exists in various grades:

Cast PMMA is a grade of PMMA formed by casting methyl methacrylate, mixed with initiators and possibly other additives into a form or mould. Cast PMMA has a high molecular weight and high purity, but cannot be recycled mechanically as it depolymerizes at a temperature close to its processing temperature.

Injection (also referred to as "Inj") and extrusion (also referred to as "XT") PMMAs are produced with co-monomers which can be ethyl-acrylate or methyl-acrylate, for example. Those co-monomers inhibit the depolymerization at low temperature so that the products can be injected or extruded. In theory, these products could be mechanically recycled. The polymers have also a lower molecular weight than Cast PMMA. Typically, only the transparent clear, post production materials are effectively mechanically recycled. Red injection grades can be mechanically recycled into red products. The other post-production products, which contain pigments, can be mechanically recycled into dark products only.

There are also PMMA grades that are cross-linked, or contain additives to improve the shock resistance.

The polymer(s) or mixtures to be depolymerized may be waste plastics of various compositions.

Typically, the polymer(s) to be depolymerized are in the form of granules, pellets, crushed particles, powder or liquids.

The resulting monomers are defined as the monomers resulting from the polymers following their depolymerization. Such resulting monomers depend on the nature of the starting polymers. Corresponding monomers typically include *methyl methacrylate* (MMA), styrene and crotonic acid, butyrolactone, (meth)acrylic acid, 2-pentenoic acid.

MMA is particularly preferred.

As used herein, the term "contaminants" refers to a range of additives that may be present in the plastics to be recycled into monomers, or degradation products formed during the depolymerization process, and whose removal is desired when depolymerizing the polymers into monomers, provided one or more identical or different contaminants may be present in the plastics to be recycled.

According to the invention, said contaminants include at least one contaminant that is not one of PET, PVC and PET- and PVC-degradation products, and are thus referred to as "non-PET- and non-PVC contaminants".

Typically, the PET- and PVC-degradation products comprise in particular terephthalic acid, dimethylterephthalate, hydrochloric acid and benzoic acid.

According to an embodiment, the non-PET- and non-PVC-contaminants include
metals,
metal oxides,
metal chlorides,
metal hydroxides,
hydrocarbons,
halogenated hydrocarbons,
alkali,
polymers such as EVA (Ethylene-Vinyl Acetate copolymer), polyethylene, rubbers, polyurethanes, polysiloxanes;
adhesives;
pigments and colorants such as mercury, cadmium, chromium, lead, selenium-based pigments;
cross-linking agents such as comonomers;
methacrylic acid, propionic acid and acetic acid;
plasticizers,
fillers,
lubricants,
biocides,
catalysts,
radical initiators,
light stabilizers,
heat stabilizers,
chain transfer agents,
anti-oxidants,
flame retarding agents,
odorizing agents,
antistatic agents,
impact modifiers,
blowing agents,
odor agents.

Typically, the non-PET- and non-PVC contaminants may include compounds referred to by the following CAS numbers : 12769-96-9 ; 1317-65-3 ; 1345-16-0 ; 51274-00-1 ; 65997-17-3 ; 68186-85-6 ; 68186-88-9 ; 68186-90-3 ; 68186-91-4 ; 68186-94-7 ; 68187-11-1 ; 68187-51-9 ; 68412-38-4 ; 71631-15-7 ; 8007-18-9 ; 58339-34-7 ; 68187-49-5 ; 8048-07-5 ; 68186-97-0 ; 61951-89-1 ; 12236-03-2 ; 164251-88-1 ; 68186-87-8 ; 68187-15-5 ; 68187-54-2 ; 80748-21-6 ; 68186-92-5 ; 54824-37-2 ; 7664-93-9 ; 7440-43-9 ; 1306-19-0 ; 7778-50-9 ; 10588-01-9 ; 1308-38-9 ; 7789-12-0 ; 1314-41-6 ; 1317-36-8 ; 7439-92-1 ; 13424-46-9 ; 7758-97-6 ; 7439-97-6 ; 1309-64-4 ; 12737-27-8 ; 12767-90-7 ; 1309-37-1 ; 1314-13-2 ; 1317-61-9 ; 13463-67-7 ; 14059-33-7 ; 12062-81-6 ; 1317-80-2 ; 18282-10-5 ; 1306-24-7 ; 1314-35-8; 3251-23-8; 7779-88-6; 10108-64-2; 7790-79-6; 7790-80-9; 7758-95-4; 7487-94-7; 7546-30-7; 10112-91-1 ; 7786-30-3; 7646-78-8; 7646-85-7; 7550-45-0; 7772-99-8 ; 16923-95-8 ; 16919-27-0 ; 13826-88-5 ; 1306-23-6 ; 7446-14-2 ; 12656-85-8 ; 1344-37-2; 1344-48-5; 7783-35-9; 1314-98-3; 7727-21-1 ; 7727-43-7; 7631-90-5; 10124-36-4 ; 7446-27-7 ; 12141-20-7 ; 13453-65-1 ; 16038-76-9 ; 24824-71-3 ; 53807-64-0 ; 10101 -66-3 ; 265647-11-8 ; 12167-74-7 ; 7631-86-9 ; 7440-21-3 ; 102184-95-2 ; 17010-21-8 ; 25808-74-6 ; 1333-86-4 ; 81-77-6 ; 89-65-6 ; 143-29-3; 198-55-0 ; 2475-46-9 ; 16294-75-0 ; 545-55-1 ; 542-83-6 ; 1319-46-6 ; 628-86-4 ; 1335-31-5 ; 471-34-1 ; 546-93-0 ; 14455-29-9 ; 25869-00-5 ; 1328-53-6 ; 14302-13-7 ; 50-00-0 ; 2832-40-8 ; 842-07-9 ; 3846-71-7 ; 25973-55-1 ; 36437-37-3 ; 149-57-5 ; 85-68-7 ; 117-81-7 ; 84-74-2 ; 53306-54-0 ; 84-66-2 ; 605-50-5 ; 84-75-3 ; 77-90-7 ; 120-55-8 ; 33703-08-1 ; 27138-31-4 ; 68-12-2 ; 50-32-8 ; 56-55-3 ; 120-12-7 ; 192-97-2 ; 193-39-5 ; 205-82-3 ; 205-99-2 ; 207-08-9 ; 218-01-9 ; 84-61-7 ; 26761-40-0 ; 10081-67-1 ; 103-23-1 ; 109-31-9 ; 128-37-0 ; 131-56-6 ; 131-57-7 ; 1709-70-2 ; 1843-05-6 ; 2082-79-3 ; 23128-74-7 ; 2440-22-4 ; 28553-12-0 ; 3147-75-9 ; 32687-78-8 ; 36443-68-2 ; 52829-07-9 ; 57-11-4; 57-13-6; 65-85-0 ; 6683-19-8 ; 70321-86-7 ; 77-89-4 ; 77-93-0 ; 77-94-1 ; 82469-79-2 ; 88-24-4 ; 41556-26-7 ; 82919-37-7 ; 103597-45-1 ; 1047-16-1 ; 106990-43-6 ; 107-22-2 ; 108-78-1 ; 108-88-3 ; 110-27-0 ; 110-33-8 ; 111-46-6 ; 115-77-5 ; 1189-08-8 ; 127-19-5 ; 141-17-3 ; 142-90-5 ; 3290-92-4 ; 4118-16-5 ; 4702-90-3 ; 4948-15-6 ; 5521-31-3 ; 614-45-9 ; 67-64-1 ; 68516-73-4 ; 6925-69-5 ; 71-43-2 ; 74-85-1 ; 79-10-7 ; 80-43-3 ; 80-62-6 ; 81-33-4 ; 81-48-1 ; 868-77-9 ; 94-28-0 ; 97-90-5 ; 980-26-7 ; 102-71-6 ; 105-64-6 ; 109-16-0 ; 111-42-2 ; 111-96-6 ; 115-11-7 ; 124172-53-8 ; 128-80-3 ; 140-88-5 ; 147315-50-2 ; 150-76-5 ; 1533-45-5 ; 23949-66-8 ; 2725-22-6 ; 3049-71-6 ; 32724-62-2 ; 5232-99-5 ; 6197-30-4 ; 6829-22-7 ; 72102-84-2 ; 732-26-3 ; 7397-62-8 ; 78-67-1 ; 82-38-2 ; 88-12-0 ; 924-42-5 ; 97-88-1 ; 99-97-8 ; 124-07-2 ; 334-48-5 ; 2475-45-8 ; 119-90-4 ; 84-65-1 ; 3333-62-8 ; 719-22-2 ; 2764-13-8 ; 4051-63-2 ; 106-10-5 ; 88-26-6 ; 514-10-3 ; 32360-05-7 ; 109-17-1 ; 3077-12-1 ; 85-69-8 ; 141-18-4 ; 10373-78-1 ; 3333-52-6 ; 22041-23-2 ; 22759-34-8 ; 1565-94-2 ; 106-74-1 ; 24817-92-3 ; 1113-92-4 ; 10024-58-5 ; 136-84-5 ; 63843-89-0 ; 23328-53-2 ; 6505-29-9 ; 28198-05-2 ; 81-39-0 ; 91-44-1 ; 5089-22-5 ; 40470-68-6 ; 2512-29-0 ; 574-93-6 ; 5395-50-6 ; 7576-65-0 ; 1017-56-7 ; 6300-37-4 ; 6250-23-3 ; 140-95-4 ; 3370-35-2 ; 3068-00-6 ; 85136-74-9 ; 3179-90-6, 56524-77-7 ; 116-85-8 ; 4991-32-6 ; 3179-89-3 ; 2872-52-8 ; 7327-69-7 ; 6358-83-4 ; 3720-97-6 ; 119-15-3 ; 730-40-5 ; 2581-69-3 ; 3883-23-6 ; 12236-29-2 ; 4913-31-9 ; 1854-26-8 ; 134-97-4 ; 3270-74-4 ; 6373-73-5 ; 6531-31-3; 13747-12-1 ; 3380-34-5; 32534-81-9; 1163-19-5 ; 25637-99-4; 101-14-4 ; 3864-99-1 ; 3896-11-5; 12236-62-3; 15793-73-4 ; 20749-68-2 ; 3089-17-6 ; 5280-68-2 ; 5280-78-4 ; 6358-85-6 ; 84632-65-5 ; 30125-47-4 ; 3905-19-9 ; 5280-80-8 ; 5580-57-4 ; 56-37-1 ; 61847-48-1 ; 79953-85-8 ; 591-50-4 ; 6041-94-7 ; 32432-45-4 ; 23739-66-4 ; 68399-99-5 ; 632-58-6 ; 569-61-9 ; 5421-66-9 ; 68391-30-0 ; 4261-72-7 ; 6359-50-8 ; 6359-45-1 ; 6320-14-5 ; 3207-12-3 ; 13301-61-6 ; 2787-91-9 ; 2465-27-2 ; 6364-35-8 ; 65907-69-9 ; 13810-83-8 ; 123-28-4 ; 29598-76-3 ; 41484-35-9; 693-36-7; 7128-64-5; 3622-84-2 ; 35325-02-1 ; 1212-08-4; 22796-14-1 ; 13676-91-0 ; 72968-71-9 ; 12671-74-8 ; 522-75-8 ; 68516-81-4 ; 15141-18-1 ; 1241-94-7 ; 26741-53-7 ; 29761-21-5 ; 31570-04-4 ; 78-51-3 ; 38613-77-3 ; 5945-33-5 ; 78-33-1 ; 115-88-8 ; 7580-37-2 ; 52221-67-7 ; 512-82-3 ; 22031-17-0 ; 115-96-8 ; 13674-87-8 ; 126-72-7 ; 124-64-1 ; 26248-87-3 ; 55566-30-8 ; 56-35-9 ; 4464-23-7 ; 75-74-1 ; 78-00-2 ; 301-04-2 ; 1072-35-1 ; 1335-32-6 ; 6838-85-3 ; 7428-48-0 ; 15245-44-0 ; 16183-12-3 ; 55-68-5 ; 100-57-2 ; 593-74-8 ; 627-44-1 ; 62-38-4 ; 103-27-5 ; 13302-00-6 ; 13864-38-5 ; 26545-49-3 ; 147-14-8 ; 603-36-1 ; 142-71-2 ; 28654-73-1 ; 139-12-8 ; 7360-53-4 ; 123-88-6 ; 17570-76-2 ; 5281-04-9 ; 24887-06-7 ; 6035-47-8 ; 65212-77-3 ; 2530-83-8 ; 1760-24-3 ; 2530-85-0 ; 65996-93-2 ; 90640-86-1 ; 67701-03-5 ; 63148-62-9 ; 70131-50-9 ; 91052-47-0 ; 68909-79-5; 4464-46-1 ; 1373399-58-6 ; 101357-30-6 ; 8002-74-2 ; 71888-89-6 ; 90640-80-5 ; 90640-81-6 ; 90640-82-7 ; 91995-15-2 ; 91995-17-4 ; 68515-50-4 ; 68515-49-1 ; 65447-77-0 ; 68411-46-1 ; 125643-61-0 ; 104810-48-2 ; 125304-04-3 ; 129757-67-1 ; 25852-37-3 ; 71878-19-8 ; 9003-08-1 ; 9003-20-7 ; 9011-05-6 ; 9011-14-7 ; 26007-43-2 ; 70198-29-7 ; 68515-51-5 ; 736150-63-3 ; 72245-46-6 ; 9011-13-6 ; 9003-63-8 ; 9003-42-3 ; 9011-15-8 ; 25087-17-6 ; 25609-74-9 ; 26655-94-7 ; 29356-88-5 ; 25768-50-7 ; 25087-18-7 ; 25719-51-1 ; 29320-53-4 ; 25719-52-2 ; 25986-80-5 ; 25639-21-8 ; 64114-51-8 ; 25189-01-9 ; 75673-26-6 ; 25213-39-2 ; 25087-19-8 ; 30525-99-6 ; 9086-85-5 ; 70624-18-9 ; 82451-48-7 ; 9002-86-2 ; 101357-16-8 ; 85507-99-9 ; 118658-98-3 ; 61790-14-5 ; 8003-05-2 ; 68512-13-0 ; CAS 79-41-4 ; CAS 64-19-7 and 81457-65-0.

The process of the invention thus allows to reduce the content of toxic ingredients in the resulting monomers. It may also lead to reduced acidity, reduced coloring in the monomers and/or reduce the corrosion risks of the recycling unit.

As used herein, the term "trapping agent" refers to agents capable of interacting with the contaminants, typically through immobilizing or degrading contaminants.

Immobilization refers to chemical or physical immobilization, involving covalent bonds or physical interactions, respectively. Such immobilization may occur in particular by adsorbing the contaminant at the surface of the trapping agent. The resulting immobilization may involve stabilization the contaminants on a solid support through a chemical reaction.

Degradation includes notably chemical reactions, acid-base neutralizations in which the contaminants react with the trapping agent so as to lead to a resulting distinct compound.

The contaminants may be immobilized with or degraded into other products which are less toxic and/or more easily extracted from the resulting monomers, so that the contaminants may be directed to waste streams which allow straightforward disposal.

The trapping agents include solid compounds like:
dolomite;
diatomaceous earth;
magnesium-, calcium-, aluminum-, strontium-, barium-, lanthanum-oxides, -hydroxides, sulfates and carbonates;
metal oxides such as zinc and iron oxides;
supported organic peroxides and inorganic peroxides;
glass fibers;
aluminum trihydroxide, as well as aluminum oxide and hydrated aluminum oxide, silica-alumina, titanium dioxide
and combinations of those materials.

Typically, said trapping agents may be chosen from dolomite, diatomeaceous earth, magnesium -oxide, -hydroxide or -carbonate, aluminum oxide or trihydrate, calcium oxide, hydroxyde or carbonate, titanium dioxide, and silica-alumina.

Typically, the recycling of PMMA contaminated by EVA by depolymerization leads to the formation of acetic acid. It was particularly found that the addition of a trapping agent for the depolymerization was able to significantly reduce the content in acetic acid in the crude MMA that was obtained following depolymerization.

Aluminium trihydroxide is typically used in solid surface materials in combination with PMMA. It was found that when these « solid surface » composites (notably composites of PMMA with aluminium trihydrate) were added to a colored PMMA containing pigments like those based on cadmium, chromium, lead or selenium, the color of the crude MMA collected at the condenser downstream of the depolymerization unit was significantly reduced.

As used herein, "supported peroxides" refer to organic peroxides diluted with a solid support such as solid particles to improve the stability of the peroxide and facilitating its handling. Such supported peroxides may typically be in the form of granules or pellet forms.

According to an embodiment, the supported organic peroxides include supported Luperox 120XL45, Luperox 101XL45, Luperox HP101XLP, Luperox 101PP20, Luperox F40KE, F40KEP, Luperox DC40P, DC40KE, DC40KEP, Luperox 231XL40, 230XL 40, NOROX^{®}DCP-40CC, NOROX^{®}DCP-40BKC, DHBP-45-IC2, Trigonox 101-50D-PD, Trigonox 101-45B-GR, Trigonox 101-45D-PD, Perkadox 14-40B-PD/GR-S, Perkadox 14-40MB-GR-S, Perkadox 14-EP40, Perkadox BC-40B-PD/GR, Perkadox BC-40K-PD, Perkadox BC-40MB-GR, Perkadox BC-EP40, Triganox 17-40B-PD/GR, Triganox 17-40MB-GR, Triganox 29-40B-PD/GR-E and Triganox 29-40MB-GR-E.
Inorganic peroxides include sodium percarbonate, sodium perborate.

### Typically:

Ca-, Mg-, Zn-based materials, dolomite, diatomaceous earth (also called diatomeous earth), supported peroxides may be used as trapping agents for recycling plastics containing EVA ;
Aluminum oxide and hydroxide, TiO₂, Glass fiber, silica-alumina, « Solid Surface » composite, AI(OH)3 ; Barium sulfate may be used as trapping agents for recycling plastics containing pigments ;
Ca-, Mg-, Zn-based materials, dolomite, diatomeous, supported peroxides may be used as trapping agents for recycling plastics containing methacrylic acid or plastics generating methacrylic acid during the depolymerization process ;
Ca-, Mg-, Zn-based materials, dolomite, diatomeous may be used as trapping agents for recycling plastics containing / generating acetic acid.
In addition to at least one non-PET or non-PVC contaminants, the plastics may also contain PET or PVC contaminants. In this case:
Ca-, Mg-, Zn-based materials, dolomite, diatomeous can be also used as (additional) trapping agents for recycling plastics further comprising PET contaminants ;
Ca(OH)₂ may be used as an additional trapping agent for recycling plastics further comprising PVC contaminants.

According to the invention, the trapping agent is contacted with the contaminated plastics that are molten during the depolymerization, whereby it can interact with the contaminants while being dispersed in the depolymerizing polymer matrix.

The trapping agent may be endogenous or exogenous to the polymers to be depolymerized.

The trapping agent is herein referred to as endogenous when it is already present in some plastics that are not currently recycled by depolymerization. In this case, both the contaminants and trapping agents are present in the plastics to be depolymerized ((ie) the primary plastics) and may interact during the depolymerization process.

According to an embodiment, the trapping agent is referred to as exogenous as it is not present in the plastics to be recycled (the "primary plastics"). The trapping agent is typically added to the plastics to be recycled.

According to a first alternative, the trapping agent may be added to the plastics to be depolymerized as a separate component.

In this first alternative, the process thus comprises the step of adding said trapping agent to the primary plastics to be recycled.

Alternatively, the trapping agent may be added as part of a secondary plastic that is added to the primary plastics to be recycled. Secondary plastics thus refers to plastics comprising an additive acting as a trapping agent for the contaminants present in the primary plastics to be recycled.

This second alternative typically applies when the trapping agent is also a plastics additive and is present as such in the secondary plastics. This second alternative allows to use such additive as a trapping agent which in turn will interact with contaminants of the primary plastics, thus allowing collection of both the contaminants and the trapping agent/additive.

According to this second alternative, the process thus comprises the step of adding a secondary plastics comprising said trapping agent to the primary plastics to be recycled.

This embodiment may be in particular illustrated by the use of PMMA wastes as secondary plastics, as a source of trapping agents.

Typically, PMMA wastes suitable as secondary plastics include:
plastics based on PMMA optionally containing EVA with sanitary grades which may contain additives such as calcium carbonate, aluminum trihydrate or TiO₂ which in turn act as trapping agent for contaminants present in the primary plastics to be recycled. including PMMA wastes;
plastics based on PMMA grades which contain titanium oxide, barium sulfate, and/or glass fibers;
plastics used for solid surface materials are based on PMMA with aluminum hydroxide as an additive. Aluminum hydroxide converts into aluminum oxide which is highly reactive and can act as a good trapping agent for pigments.

Therefore, according to an embodiment, the process of the invention comprises the step of combining the plastics to be recycled with one or more secondary plastics comprising one or more trapping agents.

The combination of PMMA wastes acting as both the primary plastics to be recycled and the source of one or more trapping agents is thus a further particular embodiment of the present invention.

In practice, some PMMA-based plastics wastes have not been recycled in combination with other plastics, such as PMMA-based plastics because their recycling generates too much solid residues or undesired degradation products. This is in particular the case for PMMA-based plastics used for "solid surfaces", PMMA-based plastics including glass fibers, or PMMA-based plastics including PVC.

According to an advantageous aspect of the invention, these PMMA-based plastics wastes can be used as secondary plastics, as a source of the trapping agent.

According to an embodiment, the depolymerizing step comprises pyrolyzing the polymers. Pyrolysis refers to the thermal decomposition of said polymers at elevated temperatures, typically comprised between 250 and 600°C, preferably between 350 and 550 °C , generally in an inert atmosphere substantially free of oxygen. Through depolymerization, the polymer decomposes to generate a stream containing the corresponding monomers and the contaminants.

« Inert atmosphere » defines an atmosphere substantially free of oxygen (less than 7 % vol). This is generally achieved by circulating a stream of nitrogen, such as a countercurrent of gaseous nitrogen.

Said depolymerization may typically be conducted in a pyrolysis reactor.

A variety of depolymerization processes and technologies are available and may be suitable, provided they involve mixing of the polymers, trapping agents and contaminants before and during the depolymerization operation.

According to an embodiment, technologies that include effective mixing by means of screw feeder, twin-screw, Auger-screw or fluid bed are generally suitable.

Among the depolymerization technologies, depolymerization in a screw extruder is preferred.

According to and embodiment, the pyrolysis reactor may be a twin-screw extruder, an Auger screw reactor.

According to an embodiment, the depolymerization is continuous, including continuous mixing and/or continuous extraction of solid residues.

According to an embodiment, the extracting step comprises separating the contaminants from the depolymerized monomers.

This extracting step typically involves a condenser downstream of the depolymerization reactor.

According to an embodiment, it comprises
- pyrolyzing the plastics containing the polymer into a pyrolysis reactor so as to generate a gas stream;
- directing the gas stream from the pyrolysis reactor to a separator configured for removing impurities from the gas stream;
- directing the gas stream from the separator to a condenser for condensing the monomer contained in the gas stream;
wherein the temperature of the gas stream in the separator is maintained strictly higher than the boiling temperature of the monomer.

The stream refers to the mixture at the outlet of the depolymerization reactor. It typically comprises the monomers and the contaminants. It can be a gas stream, it being understood that the contaminants can be in the gaseous form or dispersed therein in a solid form such as dust or particulates.

As used herein, the separator defines a vessel such as a tank allowing the removal of the side products from the monomers. Typically, the separator may have a separation chamber and an inlet duct for receiving the stream generated by the pyrolysis reactor and for leading to the separation chamber, the stream being collected in the separation chamber.

According to an embodiment, the gaseous stream may be slowed down progressively inside the separator along the inlet duct. Accordingly, the separator may have an increasing downstream cross-section.

According to an embodiment, the separator is configured to avoid mist formation and/or to force mist particles to collide and make larger droplets.

It may typically comprise a demister, to allow the removal of liquid droplets that may be present in the vapor stream. Demisters may be a mesh-type coalescer, vane pack or other structure intended to aggregate the mist into droplets that are heavy enough to separate from the vapor stream.

The separator is also preferably insulated to reduce mist formation. Typically, the temperature at the bottom of the separator may be maintained above approximately 90°C, typically above 100°C at atmospheric pressure.

The separator is also configured to separate or collect the side products such as contaminants and dust, char, waxes, mist, oligomers.

The separator may also be configured to collect the side products at the bottom of the separator.

This may be typically achieved by one or more of the following features:
According to a feature, the separator may comprise a bottom plate-free helix to allow the side products to drop to the bottom of the separator. Grids inside the helix are also advantageous in that they may force the mist particles to collide and would reduce the risk of mist containing contaminants carried away to the condenser.
According to an alternative feature, the separator may also be configured to comprise the spraying of cold liquid at the outlet duct, to reduce the temperature at the exit of the separator or in the duct to the condenser, so that the side products aggregate and remain in the separator.

The side products can be continuously removed from the separator, for example with a screw extruder.

Alternatively, they can accumulate in a removable drum at the bottom of the separator.

The separator may further comprise an evaporator to evaporate condensed monomers.

The present invention is illustrated in the following non-limiting examples given for representative purpose.

### Examples:

A twin screw with co-rotation and intermeshing type is used with high free volume in the extruder and self-cleaning property. Typically, the extruder for the pilot line is a TEX44 (having a 47mm screw diameter). But for commercial production line a TEX90 (having a 96.5mm screw diameter) could be used according to the requested feeding rate. TEX extruders are marketed by Japan Steel Works, and JSW can provide even bigger extruders, larger than the TEX90.

A pilot plant was set up, including
- The extruder TEX44;
- An electric heater, providing the heating energy, which is installed around the barrels, and thermocouples for temperature control which are also installed in the barrels;.
- The twin screws which are installed in the barrels;
- The barrels and screws are arranged by segmented method, in order to easily exchange the parts;

The material to depolymerize is fed into the twin-screw extruder at the hopper barrel by gravimetric screw feeders (measuring a weight loss). The material is melted in the melting zone, mostly by shear stress from screw and by heat; then melted material is transported to the depolymerization section.

The depolymerization section uses special barrel heaters, which can achieve a maximum temperature of 550°C.

At the depolymerization section, the polymer is depolymerized by heater energy assisted by the shear stress from screw (mechanical energy), and transformed into the MMA monomer gas which is moved to downstream equipment.

A solid separator is included at the end of the extruder, to trap any solid that would be generated by the depolymerization, such as dust and non-depolymerized PMMA, but also solids from the additives used in the process, and inorganic fillers that were in the PMMA scraps processed. A condenser condenses the gaseous MMA produced by the depolymerization, and a pump continuously withdraws crude MMA from the condenser.

The unit is also equipped with several safety devices such as pressure relief valves, pressure and temperature sensors, and the like. Demisters and desublimators can also be included in the downstream section after the extruder.

PMMA mixtures in the form of crushed particles were fed through the gravimetric feeders. Two to four independent feeders were loaded with cast, injection and/or extrusion PMMA, but also with the additives to be evaluated.

The following standard operating conditions were set up:
Flow rate of the extruder: Total constant feed rate of 50 to 100 kg/h in standard conditions

Rotation speed of the screws: 800 rotations per minute, in standard conditions

Temperature of the screw extruder: 470 °C, in standard conditions. However, the first and last barrels are operated at lower temperature. The first barrels correspond to the melting zone and the last barrel is before a connecting plate connected to the solid separator. For example, the temperature profile from first to last barrel can be 14, 21, 250, 326, 427, 469, 470, 470, 470, 470, 470, 470, 470, 470, 470, 435, 405, and 271 °C for the last flange, in a case with an extrusion material and 70 kg/h.

All the PMMA cast, extrusion and injection samples were previously crushed to a size of about 5 to 10 mm.

### Example 1 (Comparative):

Black Cast PMMA (also known as smokey cast PMMA) was processed in the TEX44 twin-screw extruder from Japan Steel Works, together with EVA polymer at a 5 wt % dosage. In this experiment a first feeder was feeding the Cast PMMA at a rate of 70 kg/h, and a second feeder was used to feed an EVA polymer at a 3.5 kg/h rate. The EVA material had the following characteristics: Melting point 91 °C (measured with ISO 3146); Vicat Softening point 66 °C (measured with ISO 306 method); 0.94 g/cm3 (measured with ISO 1183). The EVA used in this process was the same material that would be used to make gaskets to be used to substitutes PVC gaskets in PMMA cast sheet production, as described in PCT/NL2021/050687.

When EVA depolymerized, it generated acetic acid, and a polymeric residue. The acetic acid produced could lead to corrosion of the equipment, and it was necessary to provide a solution to reduce its production. The acetic acid concentration in the crude MMA collected, measured by gas chromatography, was 0.45 wt %

### Example 2 (according to the invention):

The example 1 was continued with the addition of dolomite as trapping agent, containing 45 wt % of Magnesium carbonate, and 55 wt % Calcium Carbonate. Before use, the humidity content of the additive was established at 0.43 wt %. The product was commercialized as DOLOMAG^{®}. The additive was added at a rate of 3.5 kg/h, through an additional feeder. It generated additional solid residue which was collected in the separator tank.

The crude MMA was analyzed by gas chromatography. The acetic acid content in the crude MMA was reduced to 0.26 wt %.

### Example 3 (Comparative):

Injection colored grade PMMA containing red and black particles was processed in the twin screw extruder. The scraps were produced from post-production car tail lights and decorative parts produced by injection.

The feed rate of the injection scraps was 50 kg/h, the temperature of the extruder was set to 470 °C, with a screw rotation speed of 800 rpm, and the unit operated at atmospheric pressure. The color of the crude MMA product collected was dark brown. The color was measured according to the ASTM D1500 - 07 standard method. The value recorded was L5.0 ASTM Color.

### Example 4 (according to the invention):

The comparatives example 3 was extended with the addition of Siralox, a silica-alumina, as a trapping agent, having the following characteristics (surface area : 352 m²/g, pore volume : 0.88 ml/g, and 40 wt % SiO2, 60 wt % Al₂O₃).

The solid residue was collected at the end of the screw in the separator tank.

The color of the crude MMA product collected in the condenser was a light brown, transparent liquid.

The color was measured according to the ASTM D1500 - 07 standard method. The value recorded was L3.5 ASTM color. The result showed a decrease in color intensity compared to Comparative example 3.

The gas chromatography analysis did not detect a variation of the ratios of methyl acrylate, methyl isobutyrate, ethyl acrylate to MMA, but detected a decrease of the amount of the very heavy side products.

### Example 5 (according to the invention):

The silica-alumina was replaced by a white cast PMMA containing glass fibers and adhesive. This white cast material was the residue of the production of bath tubs. In this production, the PMMA white cast sheet material containing TiO₂ as pigment, was first thermoformed to make the shaped bath tub. Then the form was consolidated by projection of glass fibers and adhesive on the back side of the bath tub. Finally, the final bath tub was cut to exact dimension. The off-cuts scraps were recovered and some glass fiber was scrapped by mechanical attrition. Then, the offcuts were crushed to less than 1 cm dimension particles containing remaining glass fiber and adhesive. These off-cuts usually had no market and had to be landfilled for proper disposal.

In this example, these contaminated bath-tub scraps had been used as an additive in the depolymerization of the colored red and black injection grade PMMA, at a rate of 15 kg/h of white cast for 50 kg/h of injection red and black PMMA.

The color of the crude MMA product collected in the condenser was a light brown, transparent liquid. The color was measured according to the ASTM D1500 - 07 standard method. The value recorded was 4.0 ASTM color. The result showed a decrease in color intensity compared to Comparative example 3. The gas chromatography analysis revealed a slight decrease in concentration of Methyl Acrylate, Ethyl Acrylate and several other impurities compared to the comparative example. However the Methyl Isobuturate content remained constant.

### Example 6 (Comparative):

Cast clear PMMA waste, without PolyEthylene (PE) film and without PolyVinylChloride (PVC) gasket, crushed to less than 1 cm particles, was fed in the extruder at a rate of 70 kg/h. EVA polymer, like in the comparative example 1, was added at a rate of 3.5 kg/h in the extruder. The screw rotation speed was 800 rpm.

The crude MMA was analyzed by Gas Chromatography. The acetic acid content was 0.47 wt %.

### Example 7 (according to the invention):

The example 6 was continued with the addition of dolomite beads as trapping agents. The dolomite beads had the following particle size distribution : 2-4.5 mm. They were commercialized by the company LHOIST as Akdolit 2G.

The dolomite had a magnesium oxide content of 31 wt %.

The additive was added at a rate of 2 kg/h through a separate feeder.

The acetic acid content was measured by gas chromatography at 0.12 wt %.

### Example 8 (according to the invention):

Example 7 was reproduced by adding the additive (dolomite beads) at a rate of 4 kg/h rate. The acetic acid content was measured by gas chromatography at 0.14 wt %. It illustrated that a dosage below 2 kg/h would be sufficient to have the trapping effect.

### Example 9 (Comparative):

A waste cast clear PMMA sample was fed at a rate of 90 kg/h. The screw rotation speed was 800 rpm.

The crude MMA collected in the condenser was analyzed. The methacrylic acid content was 1.05 wt %

### Example 10 (according to the invention):

The example 9 was continued with the addition of dolomite beads. The dolomite beads had the following particle size distribution : 2-4.5 mm. They were commercialized by the company LHOIST as Akdolit 2G.

The dolomite had a magnesium oxide content of 31 wt %.

The additive was added at a rate of 4 kg/h rate.

The crude MMA collected in the condenser was analyzed. The methacrylic acid content was 0.61 wt %.

### Example 11 (according to the invention):

Example 10 was reproduced by adding the additive at a rate of 8 kg/h rate

The methacrylic acid content was reduced to 0.40 wt %.

### Exemple 12 (Comparative) :

A smokey cast PMMA (Black) sample was used in this example. This was produced by adding a pigment in the cast formulation. The Cast PMMA was added at a rate of 90 kg/h in the extruder which was operated at 470 °C. The screw rotation speed was 800 rpm. After 4 hours of stable operation, a sample was taken. The color of the sample was a deep dark, but the crude MMA was nevertheless transparent to light.

The color of the crude MMA product collected in the condenser was a light brown, transparent liquid. The color was measured according to the ASTM D1500 - 07 standard method.

The sample was analyzed by Gas Chromatography. The Methyl Methacrylate content in the crude MMA was 96 wt % (+/- 2 %) and the Methanol content was 0.2 wt %. The acetic acid and methacrylic acid content were below 0.01 wt %.

### Example 13 (according to the invention)

The condition of the Comparative example 12 are maintained, and the additive is added through a secondary feeder.

In this example, Alumina spherical balls are used as an additive. The alumina balls have the following characteristics : Surface Area : 201 m²/g, Pore Volume : 0.92 ml/g, median pore diameter 110 angstrom. The alumina balls are added at a rate of 2 kg/h The color of the crude MMA product collected in the condenser is measured according to the ASTM D1500 - 07 standard method.

### Example 14 (according to the invention)

The condition of the Comparative example 12 are maintained, and the additive is added through a secondary feeder.

In this example, a « Solid Surface » material commercialized under the brand name « CORIAN » is used. The material is white and contains 60 wt % of aluminum trihydrate.

The Solid Surface material is crushed into pieces of 5 mm size. Then the particles are placed in trays of maximum 1 cm thickness. The trays are placed in a ventilated oven at 225 °C overnight (16 hours). In the next day, the material is cooled to room temperature, further crushed when necessary, and placed in a feeder. The material is fed at a rate of 10 kg/h and 20 kg/h in addition to the material of the comparative example 12.

The color of the crude MMA product collected in the condenser is measured according to the ASTM D1500 - 07 standard method.

### Example 15 (Comparative) :

In this example a mixed Cast and Extrusion sheets PMMA sample was used. The product was made of about 80 wt % of cast PMMA. It contained dark particles and multiple colors, fluorescent colors, but also some transparent materials.

The unit was operated at 490 °C, and at a feeding rate of 60 kg/h, and 800 rotations per minute. After 1 hr of operation, the crude MMA sample was a deep dark product. The color of the crude MMA product collected in the condenser was a light brown, transparent liquid. The color was measured according to the ASTM D1500 - 07 standard method.

The Methyl methacrylate content was measured by gas chromatography and was 91 wt % (+/- 2 %), and the methanol content was 0.64 wt %.

### Example 16 (according to the invention) :

The previous example 15 is continued with the addition of calcined diatomeous earth at a rate of 5 kg/h. For ease of operation in the feeder, this is used as lumps of about 3 mm size. The color of the crude MMA product collected in the condenser is measured according to the ASTM D1500 - 07 standard method.

## Claims

1. Process for recycling plastics comprising one or more polymers contaminated by contaminants, where at least one contaminant is other than PET, PVC or their degradation products, said process comprising the steps of:
contacting at least one solid trapping agent with the contaminated polymers to be depolymerized;
depolymerizing the polymers leading to corresponding monomers ;
whereby the non-PET and non-PVC contaminants interact with the solid trapping agents; and
continuously extracting at least partially the non-PET and non-PVC contaminants associated with the solid trapping agent from the reaction mixture.

2. Process according to claim 1 wherein the PET- and PVC-degradation products comprise terephthalic acid, dimethylterephthalate, hydrochloric acid and benzoic acid.

3. Process according to anyone of the preceding claims wherein the polymers are chosen from the group consisting in poly(methyl methacrylate) (PMMA), polystyrene (PS), polyhydroxyalcanoates (PHA) which include poly-3-hydroxybutyrate, poly-3-hydroxybutyrate-4-hydroxy-butyrate, poly-3-hydroxypropionate, polyhydroxybutyrate-valerate and polyhydroxyisobutyrate.

4. Process according to anyone of the preceding claims wherein the resulting monomers are chosen from the group consisting in *methyl methacrylate* (MMA), styrene and crotonic acid, butyrolactone, acrylic acid and 2-pentenoic acid.

5. Process according to anyone of the preceding claims wherein the non-PET- and non-PVC contaminants are chosen from the group consisting in metals, metal oxides, metal chlorides, metal hydroxides, hydrocarbons, halogenated hydrocarbons, alkali, polymers (such as EVA, polyethylene, rubbers, polyurethanes, polysiloxanes) adhesives, pigments and colorants such as cadmium, chromium, lead, selenium-based pigments, cross-linking agents such as comonomers, methacrylic acid and acetic acid; plasticizers, fillers, lubricants, biocides, catalysts and radical initiators, light stabilizers, heat stabilizers, chain transfer agents, anti-oxidants, flame retarding agents, odorizing agents, antistatic agents, impact modifiers, blowing agents, odor agents.

6. Process according to anyone of the preceding claims wherein the non-PET- and non-PVC contaminants are chosen from the group consisting in compounds referred to by the following CAS numbers 12769-96-9 ; 1317-65-3 ; 1345-16-0 ; 51274-00-1 ; 65997-17-3 ; 68186-85-6 ; 68186-88-9 ; 68186-90-3 ; 68186-91-4 ; 68186-94-7 ; 68187-11-1 ; 68187-51-9 ; 68412-38-4 ; 71631-15-7 ; 8007-18-9 ; 58339-34-7 ; 68187-49-5 ; 8048-07-5 ; 68186-97-0 ; 61951-89-1 ; 12236-03-2 ; 164251-88-1 ; 68186-87-8 ; 68187-15-5 ; 68187-54-2 ; 80748-21-6 ; 68186-92-5 ; 54824-37-2 ; 7664-93-9 ; 7440-43-9 ; 1306-19-0 ; 7778-50-9 ; 10588-01-9 ; 1308-38-9 ; 7789-12-0 ; 1314-41-6 ; 1317-36-8 ; 7439-92-1 ; 13424-46-9 ; 7758-97-6 ; 7439-97-6 ; 1309-64-4 ; 12737-27-8 ; 12767-90-7 ; 1309-37-1 ; 1314-13-2 ; 1317-61-9 ; 13463-67-7 ; 14059-33-7 ; 12062-81-6 ; 1317-80-2 ; 18282-10-5 ; 1306-24-7 ; 1314-35-8; 3251-23-8; 7779-88-6; 10108-64-2; 7790-79-6; 7790-80-9; 7758-95-4; 7487-94-7; 7546-30-7; 10112-91-1 ; 7786-30-3; 7646-78-8; 7646-85-7; 7550-45-0; 7772-99-8 ; 16923-95-8 ; 16919-27-0 ; 13826-88-5 ; 1306-23-6 ; 7446-14-2 ; 12656-85-8 ; 1344-37-2; 1344-48-5; 7783-35-9; 1314-98-3; 7727-21-1 ; 7727-43-7; 7631-90-5; 10124-36-4 ; 7446-27-7 ; 12141-20-7 ; 13453-65-1 ; 16038-76-9 ; 24824-71-3 ; 53807-64-0 ; 10101 -66-3 ; 265647-11-8 ; 12167-74-7 ; 7631-86-9 ; 7440-21-3 ; 102184-95-2 ; 17010-21-8 ; 25808-74-6; 1333-86-4; 81-77-6; 89-65-6; 143-29-3; 198-55-0 ; 2475-46-9 ; 16294-75-0 ; 545-55-1 ; 542-83-6 ; 1319-46-6 ; 628-86-4 ; 1335-31-5 ; 471-34-1 ; 546-93-0 ; 14455-29-9 ; 25869-00-5 ; 1328-53-6 ; 14302-13-7 ; 50-00-0 ; 2832-40-8 ; 842-07-9 ; 3846-71-7 ; 25973-55-1 ; 36437-37-3 ; 149-57-5 ; 85-68-7 ; 117-81-7 ; 84-74-2 ; 53306-54-0 ; 84-66-2 ; 605-50-5 ; 84-75-3 ; 77-90-7 ; 120-55-8 ; 33703-08-1 ; 27138-31-4 ; 68-12-2 ; 50-32-8 ; 56-55-3 ; 120-12-7 ; 192-97-2 ; 193-39-5 ; 205-82-3 ; 205-99-2 ; 207-08-9 ; 218-01-9 ; 84-61-7 ; 26761-40-0 ; 10081-67-1 ; 103-23-1 ; 109-31-9 ; 128-37-0 ; 131-56-6 ; 131-57-7 ; 1709-70-2 ; 1843-05-6 ; 2082-79-3 ; 23128-74-7 ; 2440-22-4 ; 28553-12-0 ; 3147-75-9; 32687-78-8 ; 36443-68-2 ; 52829-07-9 ; 57-11-4; 57-13-6 ; 65-85-0 ; 6683-19-8 ; 70321-86-7 ; 77-89-4 ; 77-93-0 ; 77-94-1 ; 82469-79-2 ; 88-24-4 ; 41556-26-7 ; 82919-37-7 ; 103597-45-1 ; 1047-16-1 ; 106990-43-6 ; 107-22-2 ; 108-78-1 ; 108-88-3 ; 110-27-0 ; 110-33-8 ; 111-46-6 ; 115-77-5 ; 1189-08-8 ; 127-19-5 ; 141-17-3 ; 142-90-5 ; 3290-92-4 ; 4118-16-5 ; 4702-90-3 ; 4948-15-6 ; 5521-31-3 ; 614-45-9 ; 67-64-1 ; 68516-73-4 ; 6925-69-5 ; 71-43-2 ; 74-85-1 ; 79-10-7 ; 80-43-3 ; 80-62-6 ; 81-33-4 ; 81-48-1 ; 868-77-9 ; 94-28-0 ; 97-90-5 ; 980-26-7 ; 102-71-6 ; 105-64-6 ; 109-16-0 ; 111-42-2 ; 111-96-6 ; 115-11-7 ; 124172-53-8 ; 128-80-3 ; 140-88-5 ; 147315-50-2 ; 150-76-5 ; 1533-45-5 ; 23949-66-8 ; 2725-22-6 ; 3049-71-6 ; 32724-62-2 ; 5232-99-5 ; 6197-30-4 ; 6829-22-7 ; 72102-84-2 ; 732-26-3 ; 7397-62-8 ; 78-67-1 ; 82-38-2 ; 88-12-0 ; 924-42-5 ; 97-88-1 ; 99-97-8 ; 124-07-2 ; 334-48-5 ; 2475-45-8 ; 119-90-4 ; 84-65-1 ; 3333-62-8 ; 719-22-2 ; 2764-13-8 ; 4051-63-2 ; 106-10-5 ; 88-26-6 ; 514-10-3 ; 32360-05-7 ; 109-17-1 ; 3077-12-1 ; 85-69-8 ; 141-18-4 ; 10373-78-1 ; 3333-52-6 ; 22041-23-2 ; 22759-34-8 ; 1565-94-2 ; 106-74-1 ; 24817-92-3 ; 1113-92-4 ; 10024-58-5 ; 136-84-5 ; 63843-89-0 ; 23328-53-2 ; 6505-29-9 ; 28198-05-2 ; 81-39-0 ; 91-44-1 ; 5089-22-5 ; 40470-68-6 ; 2512-29-0 ; 574-93-6 ; 5395-50-6 ; 7576-65-0 ; 1017-56-7 ; 6300-37-4 ; 6250-23-3 ; 140-95-4 ; 3370-35-2 ; 3068-00-6 ; 85136-74-9 ; 3179-90-6, 56524-77-7 ; 116-85-8 ; 4991-32-6 ; 3179-89-3 ; 2872-52-8 ; 7327-69-7 ; 6358-83-4 ; 3720-97-6 ; 119-15-3 ; 730-40-5 ; 2581-69-3 ; 3883-23-6 ; 12236-29-2 ; 4913-31-9 ; 1854-26-8; 134-97-4; 3270-74-4; 6373-73-5; 6531-31-3; 13747-12-1 ; 3380-34-5; 32534-81-9; 1163-19-5 ; 25637-99-4; 101-14-4 ; 3864-99-1 ; 3896-11-5; 12236-62-3; 15793-73-4 ; 20749-68-2 ; 3089-17-6 ; 5280-68-2 ; 5280-78-4 ; 6358-85-6 ; 84632-65-5 ; 30125-47-4 ; 3905-19-9 ; 5280-80-8 ; 5580-57-4 ; 56-37-1 ; 61847-48-1 ; 79953-85-8 ; 591-50-4 ; 6041-94-7 ; 32432-45-4 ; 23739-66-4 ; 68399-99-5 ; 632-58-6 ; 569-61-9 ; 5421-66-9 ; 68391-30-0 ; 4261-72-7 ; 6359-50-8 ; 6359-45-1 ; 6320-14-5 ; 3207-12-3 ; 13301-61-6 ; 2787-91-9 ; 2465-27-2 ; 6364-35-8 ; 65907-69-9 ; 13810-83-8 ; 123-28-4 ; 29598-76-3 ; 41484-35-9; 693-36-7; 7128-64-5; 3622-84-2 ; 35325-02-1 ; 1212-08-4; 22796-14-1 ; 13676-91-0 ; 72968-71-9 ; 12671-74-8 ; 522-75-8 ; 68516-81-4 ; 15141-18-1 ; 1241-94-7 ; 26741-53-7 ; 29761-21-5 ; 31570-04-4 ; 78-51-3 ; 38613-77-3 ; 5945-33-5 ; 78-33-1 ; 115-88-8 ; 7580-37-2 ; 52221-67-7 ; 512-82-3 ; 22031-17-0 ; 115-96-8 ; 13674-87-8 ; 126-72-7 ; 124-64-1 ; 26248-87-3 ; 55566-30-8 ; 56-35-9 ; 4464-23-7 ; 75-74-1 ; 78-00-2 ; 301-04-2 ; 1072-35-1 ; 1335-32-6 ; 6838-85-3 ; 7428-48-0 ; 15245-44-0 ; 16183-12-3 ; 55-68-5 ; 100-57-2 ; 593-74-8 ; 627-44-1 ; 62-38-4 ; 103-27-5 ; 13302-00-6 ; 13864-38-5 ; 26545-49-3 ; 147-14-8 ; 603-36-1 ; 142-71-2 ; 28654-73-1 ; 139-12-8 ; 7360-53-4 ; 123-88-6 ; 17570-76-2 ; 5281-04-9 ; 24887-06-7 ; 6035-47-8 ; 65212-77-3 ; 2530-83-8 ; 1760-24-3 ; 2530-85-0 ; 65996-93-2 ; 90640-86-1 ; 67701-03-5 ; 63148-62-9 ; 70131-50-9 ; 91052-47-0 ; 68909-79-5 ; 4464-46-1 ; 1373399-58-6 ; 101357-30-6 ; 8002-74-2 ; 71888-89-6 ; 90640-80-5 ; 90640-81-6 ; 90640-82-7 ; 91995-15-2 ; 91995-17-4 ; 68515-50-4 ; 68515-49-1 ; 65447-77-0 ; 68411-46-1 ; 125643-61-0 ; 104810-48-2 ; 125304-04-3 ; 129757-67-1 ; 25852-37-3 ; 71878-19-8 ; 9003-08-1 ; 9003-20-7 ; 9011-05-6 ; 9011-14-7 ; 26007-43-2 ; 70198-29-7 ; 68515-51-5 ; 736150-63-3 ; 72245-46-6 ; 9011-13-6 ; 9003-63-8 ; 9003-42-3 ; 9011-15-8 ; 25087-17-6 ; 25609-74-9 ; 26655-94-7 ; 29356-88-5 ; 25768-50-7 ; 25087-18-7 ; 25719-51-1 ; 29320-53-4 ; 25719-52-2 ; 25986-80-5 ; 25639-21-8 ; 64114-51-8 ; 25189-01-9 ; 75673-26-6 ; 25213-39-2 ; 25087-19-8 ; 30525-99-6 ; 9086-85-5 ; 70624-18-9 ; 82451-48-7 ; 9002-86-2 ; 101357-16-8 ; 85507-99-9 ; 118658-98-3 ; 61790-14-5 ; 8003-05-2 ; 68512-13-0 ; CAS 79-41-4 ; CAS 64-19-7and 81457-65-0.

7. Process according to anyone of the preceding claims wherein the trapping agents are chosen from
dolomite;
diatomaceous earth;
magnesium-, aluminium-, strontium-, barium-, lanthanum-oxides, -hydroxides, sulfates and -carbonates, in particular -oxides and carbonates;
metal oxides such as zinc and iron oxides;
supported organic peroxides and inorganic peroxides;
glass fibers;
Aluminum trihydroxide (used in solid surface materials in combination with PMMA), as well as aluminum oxide and hydrated aluminum oxide,
Silica-alumina, titanium dioxide
and combinations of those materials.

8. Process according to anyone of the preceding claims where the trapping agent is:
added to the plastics to be recycled as a separate component or as an additive of a secondary plastics added to the plastics to be recycled.

9. Process according to claim 7 wherein the supported organic peroxides are chosen from supported Luperox 120XL45, Luperox 101XL45, Luperox HP101XLP, Luperox 101PP20 (less preferred), Luperox F40KE, F40KEP, Luperox DC40P, DC40KE, DC40KEP, Luperox 231XL40, 230XL 40, NOROX^{®}DCP-40CC, NOROX^{®}DCP-40BKC, DHBP-45-IC2, Trigonox 101-50D-PD, Trigonox 101-45B-GR, Trigonox 101-45D-PD, Perkadox 14-40B-PD/GR-S, Perkadox 14-40MB-GR-S, Perkadox 14-EP40, Perkadox BC-40B-PD/GR, Perkadox BC-40K-PD, Perkadox BC-40MB-GR, Perkadox BC-EP40, Triganox 17-40B-PD/GR, Triganox 17-40MB-GR, Triganox 29-40B-PD/GR-E, Triganox 29-40MB-GR-E.

10. Process according to claim 7 or 9 wherein the supported organic peroxides are in granules or pellet forms.

11. Process according to anyone of the preceding claims wherein the non-PET and non-PVC contaminants associated with the solid trapping agent are collected in the form of dust, char, waxes, mist or oligomers.

12. Process according to anyone of the preceding claims wherein the depolymerizing step is conducted by pyrolizing the plastics into a pyrolysis reactor so as to generate a stream containing the monomers and the contaminants.

13. Process according to anyone of the preceding claims wherein the extracting step comprises removing said contaminants from the stream in a separator downstream of the reactor,
directing the monomer gas stream from the separator to a condenser for condensing the monomers contained in the stream;
wherein, in the separator, the temperature of the stream is maintained strictly higher than the boiling temperature of the monomer.

14. The process according to claim 13 wherein the pyrolysis reactor is a twin-screw extruder, an Auger screw reactor

15. The process according to anyone of claims 13 or 14, wherein the separator has a separation chamber and an inlet duct for receiving the stream generated by the pyrolysis reactor and for leading to the separation chamber, the stream being collected in the separation chamber.

16. The process according to anyone of claims 13 to 15 wherein the stream is slowed down progressively inside the separator along the inlet duct.
